# EUROPEAN PATENT SPECIFICATION

(11) **EP 2 376 407 B1**
(45) Date of publication and mention of the grant of the patent: **05.07.2017**
(21) Application number: 09831861.1
(22) Date of filing: 27.11.2009
(51) Int. Cl.: C07C 13/62, C09K 11/06, H01L 51/50, C07D 471/04, C07C 25/22, C07C 43/21, C07C 43/275, C07C 205/11, C07C 255/52, H05B 33/14, H01L 51/00

(54) **Radio-opaque bioactiveglass materials**
Röntgendichte bioaktive Glasmaterialien
Matériaux en verre bioactif radio-opaque

(30) Priority: 10.12.2008 JP 2008314391
(43) Date of publication of application: 19.10.2011
(73) Proprietor: Canon Kabushiki Kaisha, Tokyo 146-8501 (JP)
(72) Inventor: NISHIURA, Chiaki, Tokyo 146-8501 (JP); KAMATANI, Jun, Tokyo 146-8501 (JP); TOMONO, Hiroyuki, Tokyo 146-8501 (JP)
(74) Representative: TBK
(86) International application number: PCT/JP2009/070359
(87) International publication number: WO 2010/067757

(56) References cited:
- WO-A1-2008/059713
- WO-A1-2008/146720
- JP-A- 2002 069 044
- JP-A- 2009 280 522

## Description

### TECHNICAL FIELD

The present invention relates to a benzoindenochrysene compound and an organic light-emitting device using the same.

### BACKGROUND ART

An organic light-emitting device is an electronic device in which a thin film comprising a fluorescent organic compound or a phosphorescent organic compound is disposed and supported between an anode and a cathode. With excitons generated from a fluorescent compound or a phosphorescent compound in accordance with injection of holes and electrons to each electrode, an organic light-emitting device emits light as the excitons relax down to a ground state.

Recently, a significant progress has been made relating to an organic light-emitting device. The characteristic feature includes that high luminance, a variety of emission wavelengths and a high-speed response can be obtained at a low voltage and also a thin and lightweight light-emitting device can be produced. For these reason, application of an organic light-emitting device in a broad and diverse range has been suggested.

At a practical level, however, optical output with more improved luminance or higher conversion efficiency is required. In addition, there are still a lot of problems associated with durability, for example, time-dependent change due to use for a long period of time or degradation due to atmospheric gas including oxygen, moisture or the like. Furthermore, for an application in a full-color display and the like, light emission of red, green, and blue colors with good color purity is required, but it cannot be said that such needs are completely met at the present moment.

To solve the problems described above, specifically problems like emission efficiency, luminance, color purity, durability and the like, use of various materials has been proposed. In Japanese Patent Application Laid-Open Nos. H10-294177 and 2005-53806, a compound having a naphthofluorene skeleton in which a naphthalene ring is fused with a fluoranthene skeleton is disclosed, and its use as a guest for a light-emitting layer which emits blue or green color is described.

### DISCLOSURE OF THE INVENTION

The present invention is to solve the problems described above. An object of the present invention is to provide an organic light-emitting device which has light emission with high efficiency, high luminance and good color purity, as well as durability.

As a result of intensive studies to solve the problems described above, the present inventors have accomplished the present invention. Specifically, the benzoindenochrysene compound of the present invention is a compound that is represented by the following general formula (1).

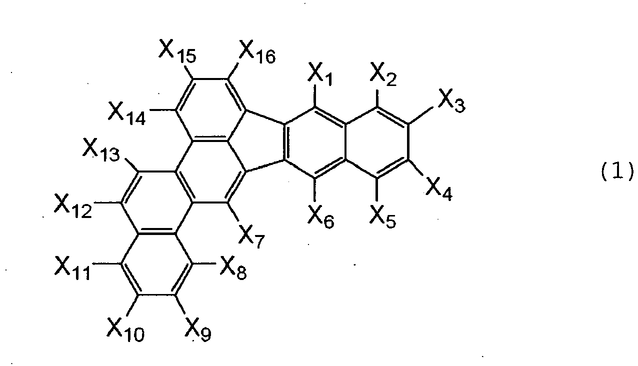

In the Formula (1), X₁, X₂, X₃, X₄, X₅, X₆, X₇, X₈, X₉, X₁₀, X₁₁, X₁₂, X₁₃, X₁₄, X₁₅, and X₁₆ each represent, independently of one another, a hydrogen atom, a halogen atom, a cyano group, a nitro group, a substituted or unsubstituted alkyl group, a substituted or unsubstituted alkoxy group, a substituted or unsubstituted alkenyl group, a substituted or unsubstituted alkynyl group, a substituted or unsubstituted aralkyl group, a substituted amino group, a substituted or unsubstituted aryl group, a substituted or unsubstituted heterocyclic group, or a substituted or unsubstituted aryloxy group.

According to the present invention, an organic light-emitting device which has light emission with high efficiency, high luminance and good color purity, as well as durability, can be provided.

### BRIEF DESCRIPTION OF THE DRAWING

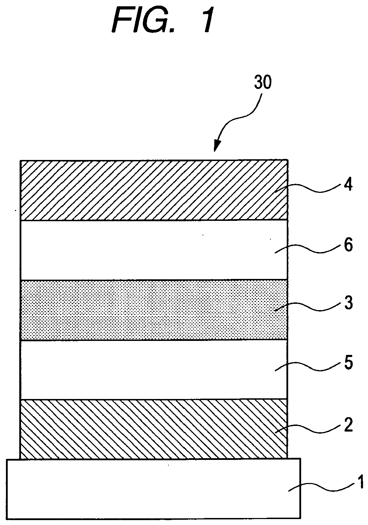

FIG. 1 is a schematic cross-sectional view illustrating a constitution of an organic light-transmitting device according to an embodiment of the present invention.

### BEST MODE FOR CARRYING OUT THE INVENTION

Herein below, the present invention will be described in detail.

First, the benzoindenochrysene compound of the present invention will be described in detail. The benzoindenochrysene compound of the present invention is represented by the following general formula (1).

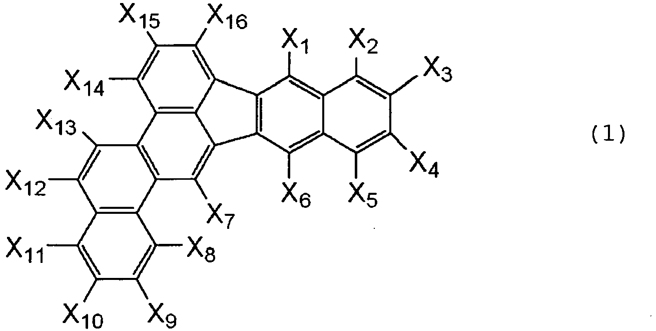

In the formula (1), X₁, X₂, X₃, X₄, X₅, X₆, X₇, X₈, X₉, X₁₀, X₁₁, X₁₂, X₁₃, X₁₄, X₁₅, and X₁₆ each represent, independently of one another, a hydrogen atom, a halogen atom, a cyano group, a nitro group, a substituted or unsubstituted alkyl group, a substituted or unsubstituted alkoxy group, a substituted or unsubstituted alkenyl group, a substituted or unsubstituted alkynyl group, a substituted or unsubstituted aralkyl group, a substituted amino group, a substituted or unsubstituted aryl group, a substituted or unsubstituted heterocyclic group, or a substituted or unsubstituted aryloxy group.

As a halogen atom that is represented by X₁ to X₁₆, a fluorine, a chlorine, a bromine, an iodine and the like can be mentioned. However, the present invention is not limited thereto.

As an example of an alkyl group that is represented by X₁ to X₁₆, a methyl group, an ethyl group, an n-propyl group, an iso-propyl group, an n-butyl group, an iso-butyl group, a sec-butyl group, a tert-butyl group, an n-pentyl group, a neopentyl group, an n-hexyl group, an n-octyl group, an n-decyl group, an n-dodecyl group, a cyclopropyl group, a cyclobutyl group, a cyclopentyl group, a cyclohexyl group, a norbornyl group, an adamantyl group and the like can be mentioned. However, the present invention is not limited thereto.

As an example of an alkoxy group that is represented by X₁ to X₁₆, a methoxy group, an ethoxy group, a propoxy group and the like can be mentioned. However, the present invention is not limited thereto.

As an example of an alkenyl group that is represented by X₁ to X₁₆, a vinyl group, a propenyl group, a butenyl group, a phenylvinyl group, a diphenylvinyl group and the like can be mentioned. However, the present invention is not limited thereto.

As an example of an alkynyl group that is represented by X₁ to X₁₆, an ethynyl group, a propynyl group, a butynyl group, a phenethynyl group and the like can be mentioned. However, the present invention is not limited thereto.

As an example of an aralkyl group that is represented by X₁ to X₁₆, a benzyl group, a phenethyl group and the like can be mentioned. However, the present invention is not limited thereto.

As an example of a substituted amino group that is represented by X₁ to X₁₆, a dimethylamino group, a diethylamino group, a dibenzylamino group, a diphenylamino group, a ditrylamino group, a ditertiary butylamino group, a dianisolyl amino group, a carbazolyl group and the like can be mentioned. However, the present invention is not limited thereto.

As an example of an aryl group that is represented by X₁ to X₁₆, a phenyl group, a naphthyl group, an azulenyl group, an acenaphthylenyl group, an indacenyl group, a biphenyl group, a fluorenyl group, an anthryl group, a phenanthryl group, a pyrenyl group, a chrysenyl group, a benzofluorenyl group, a tetraphenyl group, a naphthacenyl group, a triphenylenyl group, a fluoranthenyl group, a pycenyl group, a pentacenyl group, a perylenyl group, a benzofluoranthenyl group, a naphthofluoranthenyl group, a benzo indeno chrysenyl group and the like can be mentioned. However, the present invention is not limited thereto.

As an example of a heterocyclic group that is represented by X₁ to X₁₆, a thienyl group, a pyrrolyl group, a pyridyl group, a bipyridyl group, an oxazolyl group, an oxadiazolyl group, a thiazolyl group, a thiadiazolyl group, a terthienyl group, a carbazolyl group, an acridinyl group, a phenanthrolyl group and the like can be mentioned. However, the present invention is not limited thereto.

As an example of an aryloxy group that is represented by X₁ to X₁₆, a phenoxy group and the like can be mentioned. However, the present invention is not limited thereto.

As an example of a substituent which the above described alkyl group, alkoxy group, alkenyl group, alkynyl group, aralkyl group, aryl group, heterocyclic group and aryloxy group may further have, there are included an alkyl group such as a methyl group, an ethyl group, a propyl group, an iso-propyl group, and a tert-butyl group, an aryl group such as a phenyl group, a naphthyl group, and a biphenyl, a heterocyclic group such as a thienyl group, a pyrrolyl group, and a pyridyl group, a substituted amino group such as a dimethylamino group, a diethylamino group, a dibenzylamino group, a diphenylamino group, a ditrylamino group, and a dianisolyl amino, an alkoxy group such as a methoxy group, an ethoxy group, and a propoxy group, an aryloxy group such as a phenoxy group, a halogen atom such as a fluorine, a chlorine, a bromine, an iodine, a cyano group, and a nitro group. However, the present invention is not limited thereto.

The benzoindenochrysene compound that is represented by the Formula (1) is preferably a compound represented by the following Formula (2).

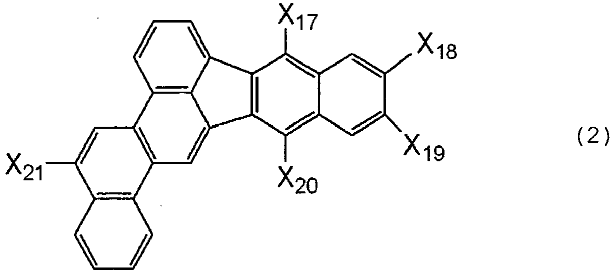

In the Formula (2), X₁₇, X₁₈, X₁₉, X₂₀, and X₂₁ each represent, independently of one another, a hydrogen atom or a substituted or unsubstituted aryl group.

As an examples of an aryl group that is represented by X₁₇ to X₂₁, a phenyl group, a naphthyl group, an azulenyl group, an acenaphthylenyl group, an indacenyl group, a biphenyl group, a fluorenyl group, an anthryl group, a phenanthryl group, a pyrenyl group, a chrysenyl group, a benzofluorenyl group, a tetraphenyl group, a naphthacenyl group, a triphenylenyl group, a fluoranthenyl group, a pycenyl group, a pentacenyl group, a perylenyl group, a benzofluoranthenyl group, a naphthofluoranthenyl group, a benzo indeno chrysenyl group and the like can be mentioned. However, the present invention is not limited thereto.

As a substituent which the above described aryl group may further have, there are included an alkyl group such as a methyl group, an ethyl group, a propyl group, an iso-propyl group, and a tert-butyl group and an aryl group such as a phenyl group, a naphthyl group, and a biphenyl group can be mentioned. However, the present invention is not limited thereto.

As one way of extending the lifetime of an organic light-emitting device, improving the emission efficiency can be mentioned. When the emission efficiency is improved, since the voltage that is applied to the device for obtaining light emission with a specific luminance can be decreased, not only power consumption can be reduced, but also degradation of the device due to carrier conduction can be reduced. Consequently, the lifetime of the organic light-emitting device can be extended.

As another way of extending the lifetime of an organic light-emitting device, improving the emission quantum yield of a light-emitting material itself can be included. Here, the present inventors measured emission quantum yield in dilute solution of the benzoindenochrysene compound of the present invention and naphthofluoranthene of the prior art (dilute toluene solution with a concentration of 10⁻⁶ mol/l). As a result, data shown in Table 1 was obtained. Incidentally, the quantum yields in Table 1 are relative quantum yield when the quantum yield of naphthofluoranthene of the prior art is set to 1.

**(Table 1)**

| Compound | Structural formula | Relative quantum yield |
|---|---|---|
| Naphthofluoranthene | 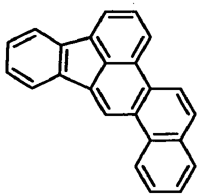 | 1.00 |
| B-1 | 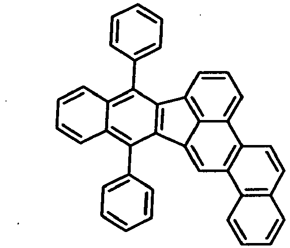 | 2.22 |
| E-25 | 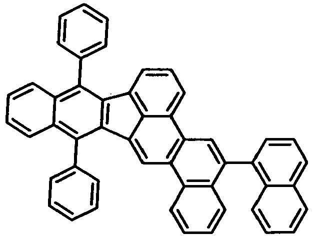 | 2.32 |
| B-7 | 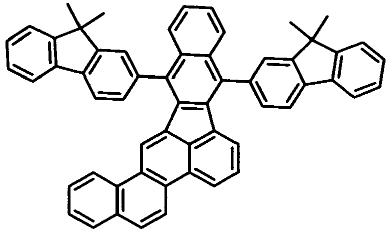 | 2.32 |
| E-28 | 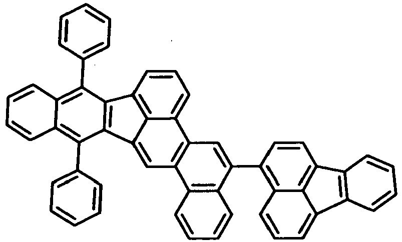 | 2.41 |

As shown in Table 1, the emission quantum yield of the benzoindenochrysene compound of the present invention is higher than that of naphthofluoranthene of the prior art. Therefore, by using the benzoindenochrysene of the present invention as a guest for a light-emitting layer, for example, the emission efficiency of an organic light-emitting device can be improved.

Meanwhile, emission maximum wavelengths for the benzoindenochrysene compounds of the present invention are shown in Table 2.

**(Table 2)**

| Compound | Structural formula | Emission maximum wavelength |
|---|---|---|
| Naphthofluoranthene | 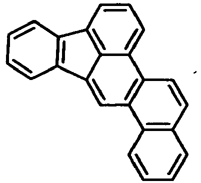 | 420 nm |
| Benzo[k] fluoranthene | 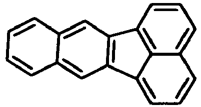 | 410 nm |
| B-1 | 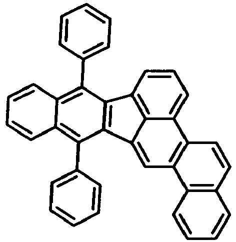 | 430 nm |
| E-30 | 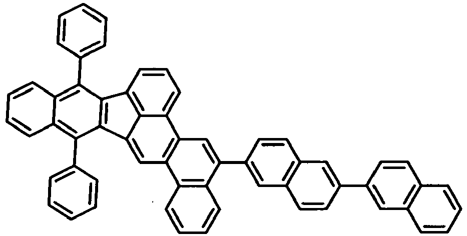 | 443 nm |
| E-34 | 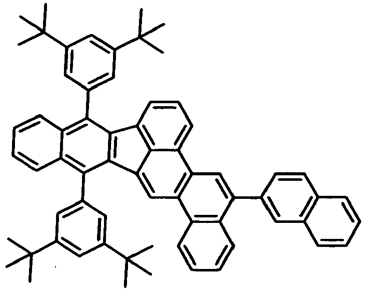 | 445 nm |

The benzoindenochrysene compounds shown in Table 2 are representative examples. With respect to the benzoindenochrysene compound of the present invention, the emission maximum wavelength is 430 nm even for those having a short wavelength. By further introducing a substituent group, shift to a longer wavelength can be achieved. Since the emission maximum wavelength which is suitable for a blue light-emitting material is within the range of 440 nm to 480 nm, in terms of the use as a blue light-emitting material, it is sufficient to increase the emission maximum wavelength by about 10 nm or less. In this case, the increase of the emission maximum wavelength by about 10 nm can be achieved by introduction of a substituent group having a relatively small size, for example, an aryl group including a naphthyl group and the like.

Meanwhile, since the emission maximum wavelengths of benzofluoranthene and naphthofluoranthene, which are analogous compounds, are 410 nm and 420 nm, respectively, if it is desired to use them as blue light-emitting materials, the emission maximum wavelength needs to be increased by about 30 nm at the maximum. In this case, in order to increase the emission maximum wavelength by about 30 nm or so, since a substituent group having relatively narrow energy gap needs to be introduced, the types of the substituent groups which can be introduced are limited.

The benzoindenochrysene compound of the present invention preferably consists of hydrocarbon only. This is because, when the compound itself consists of hydrocarbon only, purification is easier as compared to a compound containing a heteroatom. The reason for easier purification includes that, in general a molecule containing a heterocycle or a heteroatom is highly polar because of having a lone pair of electrons and may easily incorporate ionic impurities as compared to a molecule consisting of hydrocarbon only. It has been known that when an impurity is incorporated into a material which constitutes an organic light-emitting device, the performance of the device is significantly impaired due to the presence of such an impurity. For such reasons, in terms of a use as a light-emitting material in particular, it is preferable that the compound concerned consists only of hydrocarbon which hardly incorporates any impurity. Furthermore, since the electron-donating property or electron-withdrawing property of a heterocyclic group or a heteroatom is stronger than that of hydrocarbon, the energy value or energy gap of HOMO-LUMO of the molecule is significantly large or small. Meanwhile, the energy level (HOMO, LUMO) of a compound consisting of hydrocarbon only can be varied symmetrically depending on the conjugation length of the molecule, and therefore it is also advantageous in that a compound having desired energy level and energy gap can be easily designed.

Molecular designing and creation of the benzoindenochrysene compound of the present invention were accomplished in view of the aspects described above.

Next, a method for synthesizing the benzoindenochrysene compound of the present invention will be described. The benzoindenochrysene compounds that are represented by the Formula (1) and the Formula (2) are synthesized according to Scheme 1 to 4 shown below.
(1) Diketonization of chrysene derivative 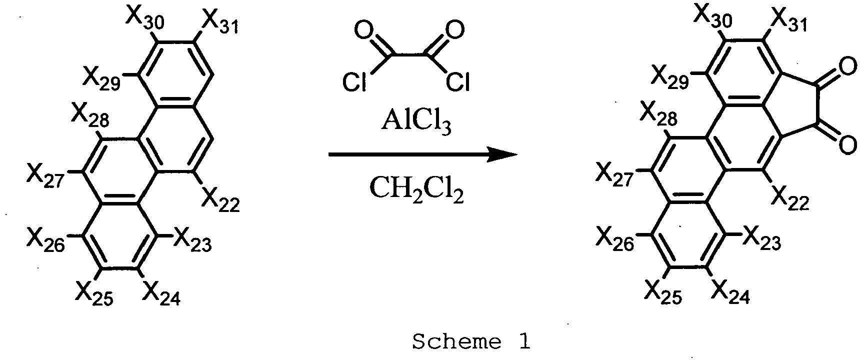
(2) Formation of cyclopentadienone in chrysene derivative 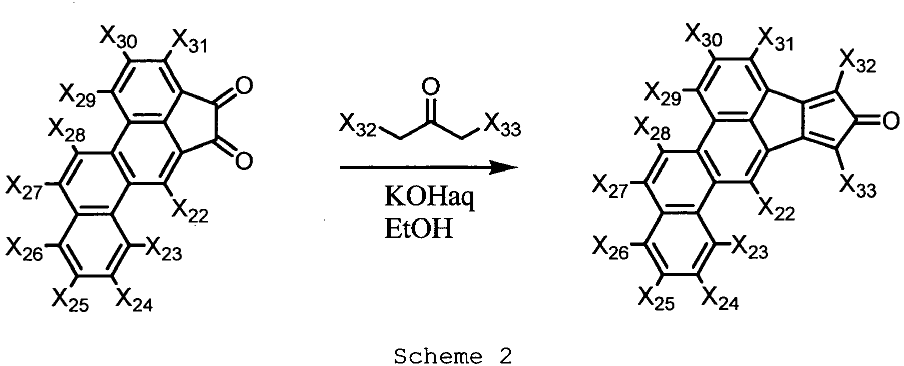
(3) Formation of benzoindenochrysene skeleton 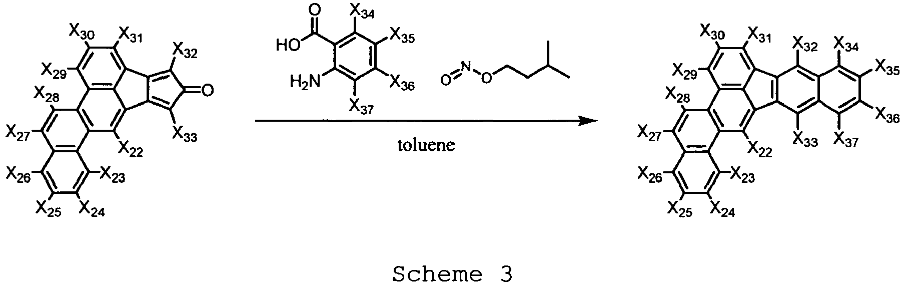
(4) Bromination of benzoindenochrysene derivative, Suzuki coupling reaction

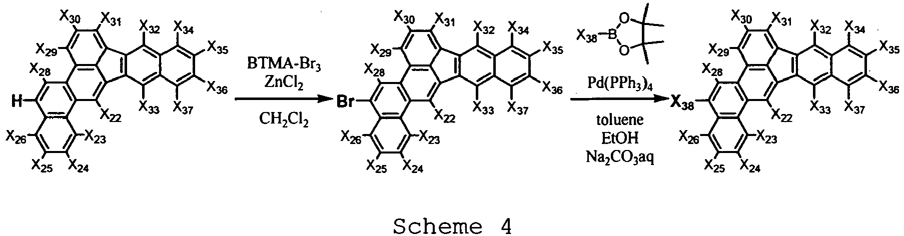

In Scheme 1, by appropriately selecting a chrysene derivative as a raw material, desired substituent groups can be introduced into X₂₂ to X₃₁.

In Scheme 2, by appropriately selecting an acetone derivative, it is possible to introduce desired substituent groups to X₃₂ and X₃₃. In this regard, when the substituent groups of X₃₂ and X₃₃ are aryl groups, that is, X₁₇ and X₂₀ in the Formula (2) are aryl groups, a structure in which the aryl group as a substituent group is orthogonal to the benzoindenochrysene skeleton as a main skeleton is formed. This is suggested when structure optimization is carried out based on molecular orbital calculation and it may be attributable to steric repulsion between hydrogen atoms of the benzoindenochrysene skeleton and the aryl group. Thus, by having the substituent group which is orthogonal to the main skeleton, concentration quenching caused by association among molecules, etc. is inhibited and as a result, doping at high concentration can be achieved.

In Scheme 3, by appropriately selecting an anthranilic acid derivative, it is possible to introduce desired substituent groups to X₃₄ to X₃₇. Specifically, according.to Scheme 3, various substituent groups can be introduced into X₁₈ and X₁₉ in the Formula (2). Furthermore, by using an anthranilic acid derivative in which any one of X₃₅ and X₃₆ is a halogen atom, a halogen atom can be introduced to any one of X₁₈ and X₁₉ in the Formula (2). When any one of X₁₈ and X₁₉ in the Formula (2) is a halogen atom, a substituent group can be easily introduced via Suzuki coupling reaction to a position at which substitution with the halogen atom is carried out, and as a result, various compounds can be synthesized. In addition, based on Suzuki coupling reaction, when a substituent group is introduced to any one of X₁₈ and X₁₉ in the Formula (2), desired physical properties such as improved emission efficiency and inhibited concentration quenching can be obtained depending on the type of a boronic acid derivative that is selected.

Furthermore, when a substituent group is introduced to any one of X₁₈ and X₁₉ in the Formula (2), emission maximum wavelength can be increased relatively significantly. Specific examples are shown in the following Table 3.

**(Table 3)**

| Compound | Structural formula | Emission maximum wavelength |
|---|---|---|
| B-1 | 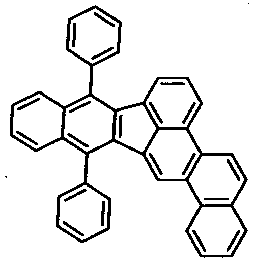 | 430 nm |
| E-25 | 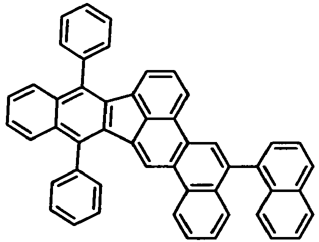 | 435 nm |
| E-13 | 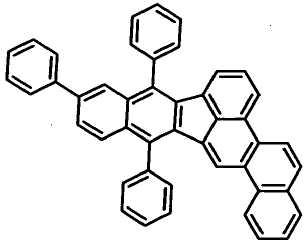 | 438 nm |

The compound E-25, in which a naphthyl group is introduced to X₂₁ position of the compound B-1 of the Formula (2), has a wavelength that is increased by about 5 nm as compared to the compound B-1. On the other hand, when a phenyl group, which has narrower energy gap as compared to a naphthyl group, is introduced to X₁₈ position of the compound B-1 of the Formula (2), the wavelength is increased by 8 nm. Thus, by introducing a substituent group to any one of X₁₈ and X₁₉ of the Formula (2), the emission maximum wavelength can be increased more largely as compared to a case in which the introduction is made to X₂₁ position. Thus, when it is intended to increase wavelength more largely, it is preferable that a substituent group is introduced into any one of X₁₈ and X₁₉ of the Formula (2).

Meanwhile, in Scheme 3, when X₂₇ is a hydrogen atom, a substituent group can be further introduced into X₂₇ position by following the method shown in Scheme 4. Specifically, by appropriately selecting a boronic acid derivative, it is possible to introduce a substituent group that is represented by X₃₈ to X₂₁ of the Formula (2). In this case, since the halogenation reaction shown in Scheme 4 has regioselectivity, a difficult purification process such as removal of positional substituent group is not required. In addition, by reacting the benzoindenochrysene compound in which a halogen atom has been introduced by the halogenation reaction with various boronic acid derivatives using Suzuki coupling reaction, various compounds can be easily synthesized. Furthermore, when a substituent group is introduced into X₂₁ of the Formula (2), desired physical properties such as improved emission efficiency and inhibited concentration quenching can be obtained depending on the type of the substituent group.

Furthermore, when an aryl group is introduced into X₂₁ of the Formula (2), the plane of the main skeleton (benzoindenochrysene skeleton) and the plane of the substituent group (aryl group) do not exist on the same plane. This is suggested when structure optimization is carried out based on molecular orbital calculation and it may be attributable to steric repulsion between hydrogen atoms in the benzoindenochrysene skeleton and the aryl group. Consequently, molecular association among the chrysene moieties that are relatively highly planar is inhibited, and as a result, the compound becomes a material which is even more difficult to be subjected to concentration quenching.

By following Scheme 1 to Scheme 4 described above, the benzoindenochrysene compound of the present invention can be synthesized. However, the above Scheme 1 to Scheme 4 are only one of specific examples of the synthetic method, and the present invention is not limited thereto.

Herein below, specific examples of the benzoindenochrysene compound of the present invention are enumerated. However, the present invention is not limited thereto.

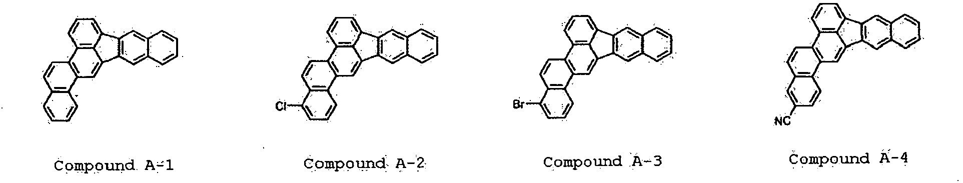

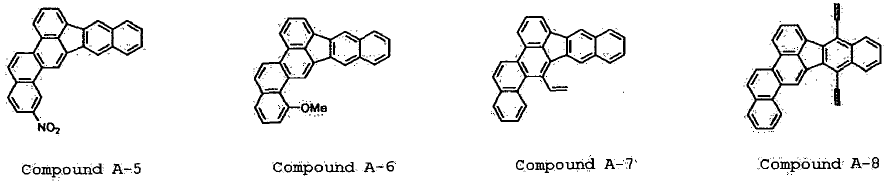

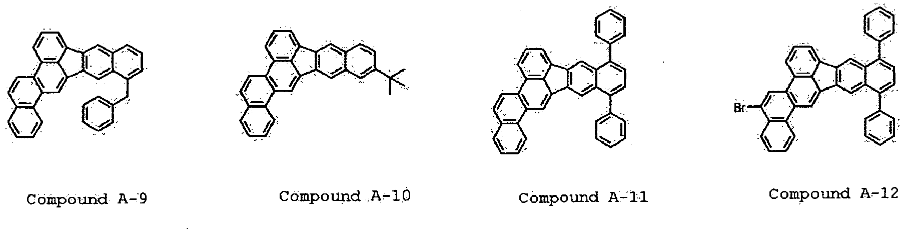

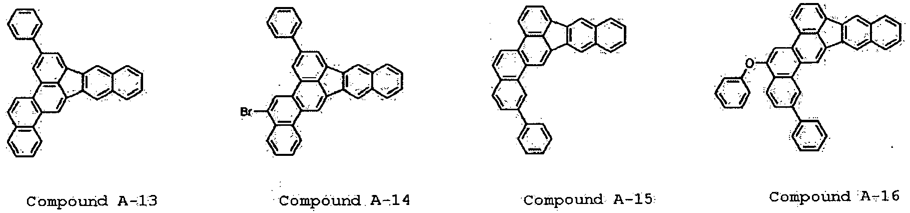

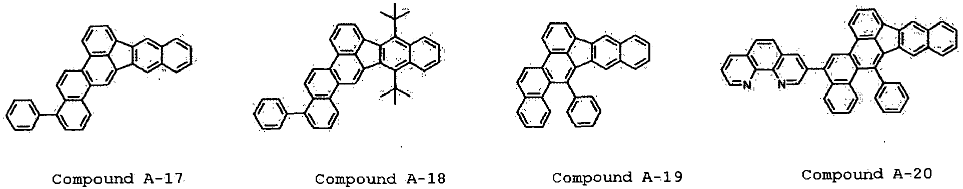

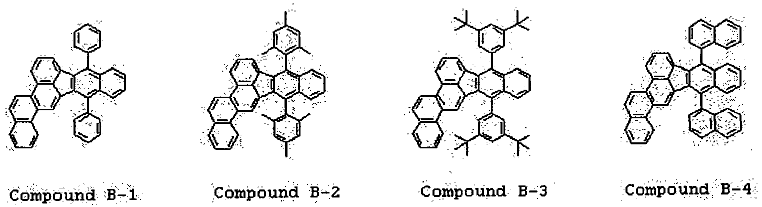

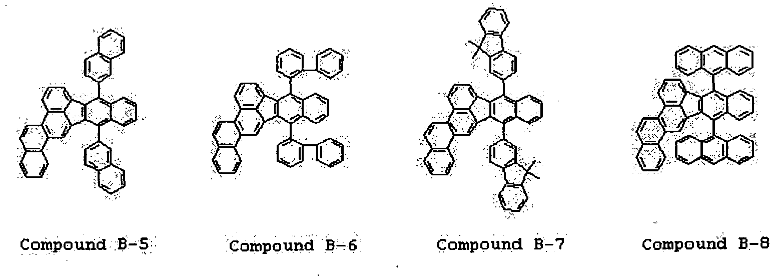

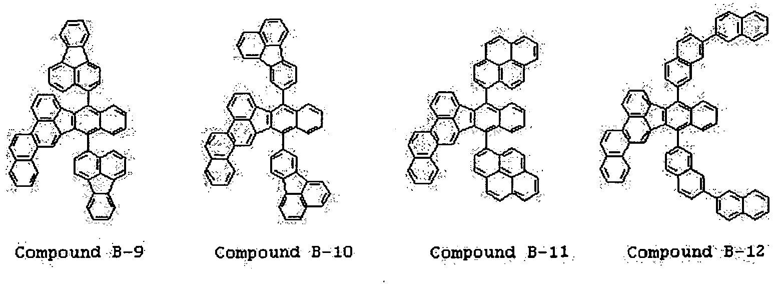

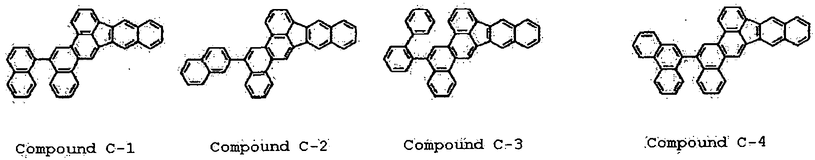

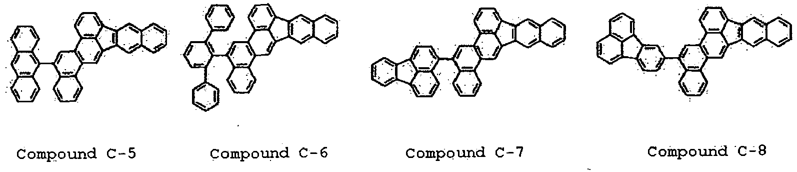

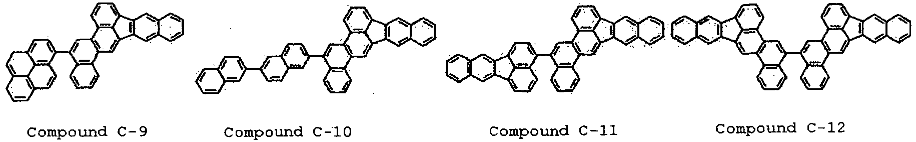

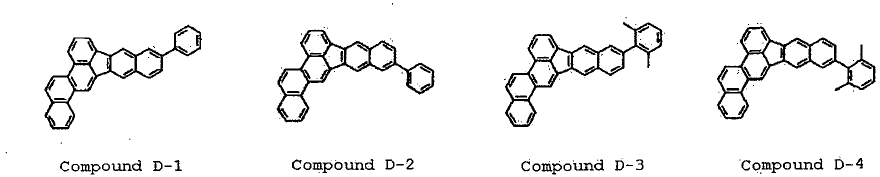

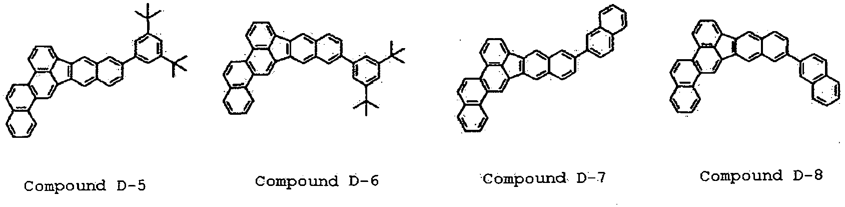

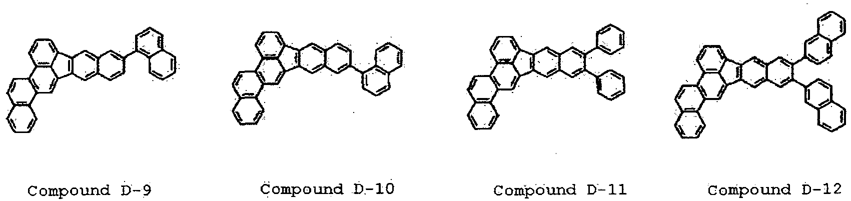

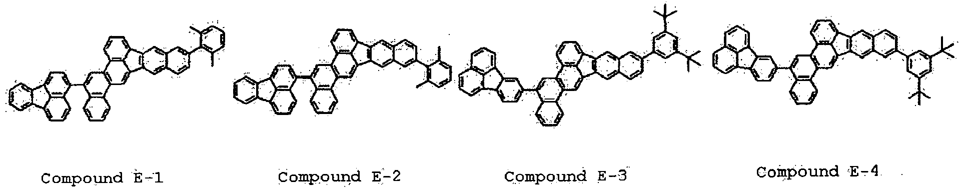

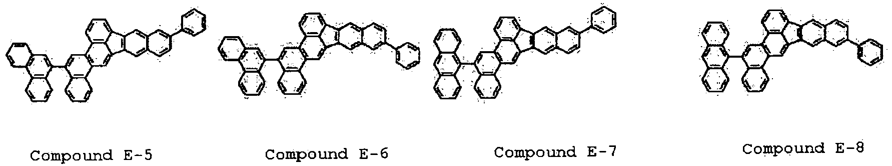

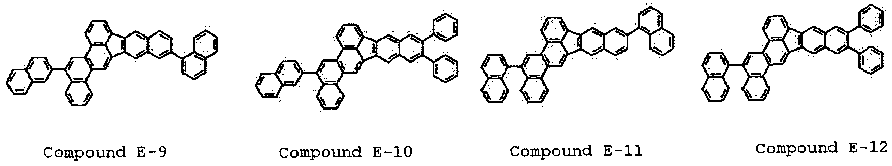

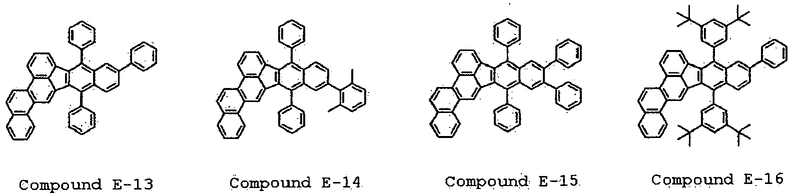

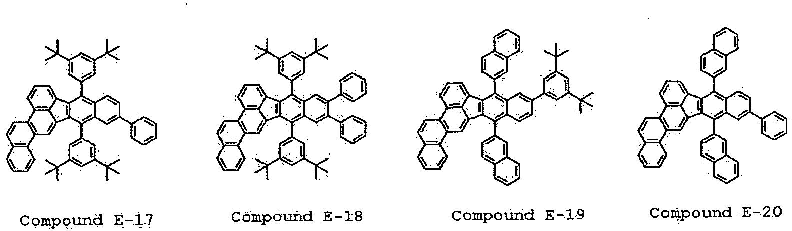

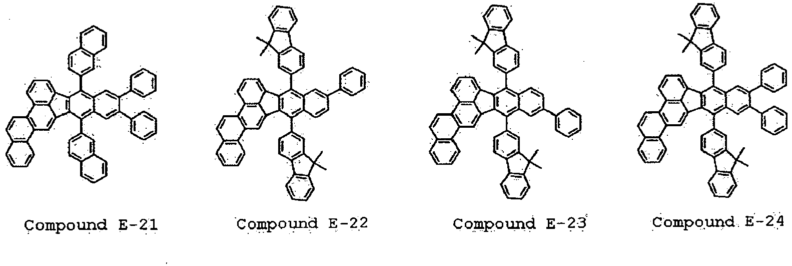

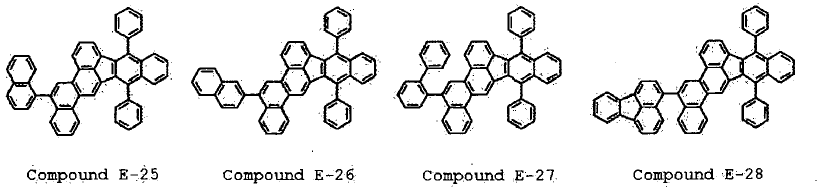

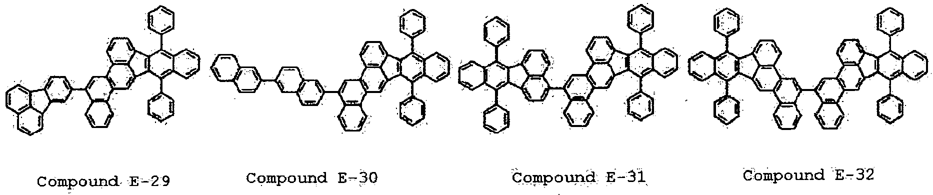

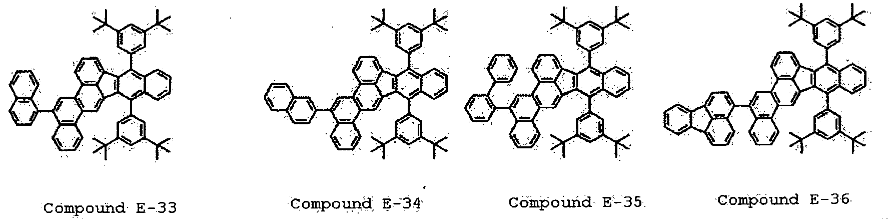

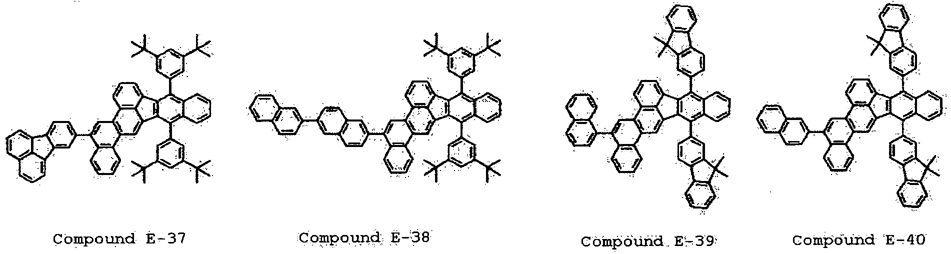

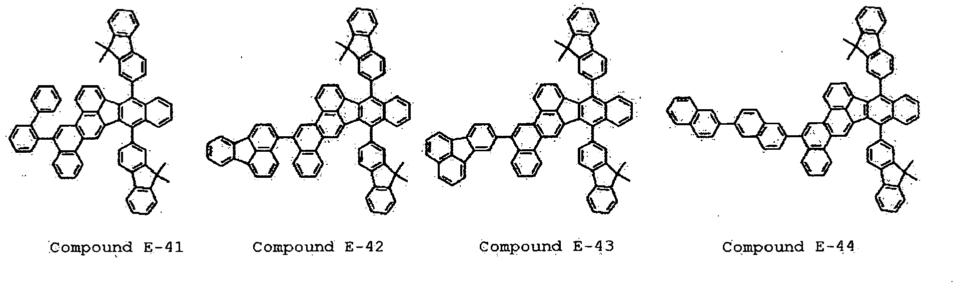

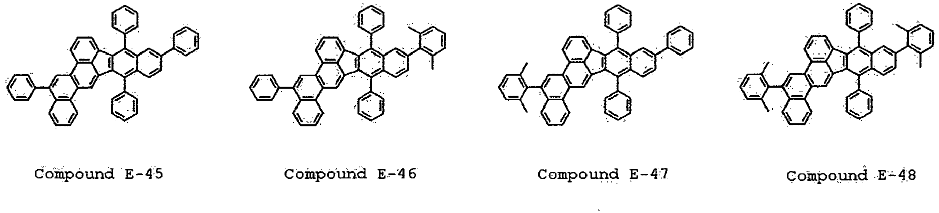

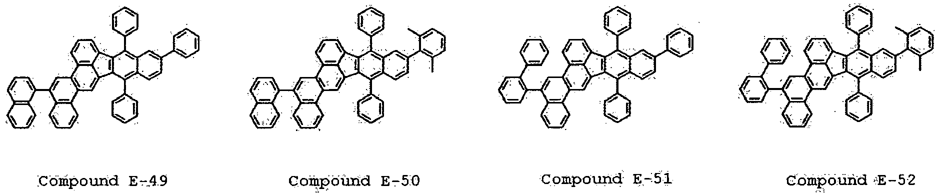

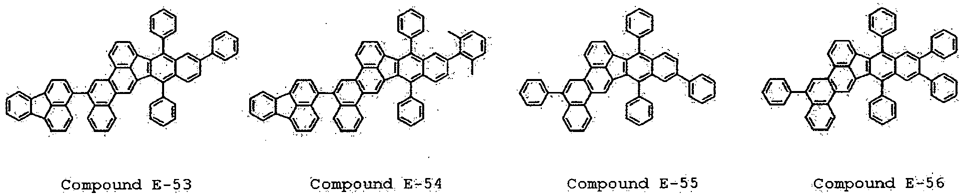

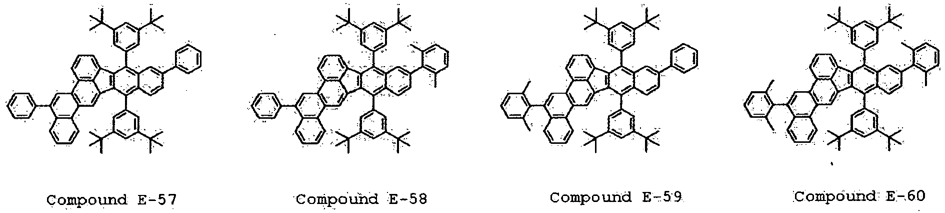

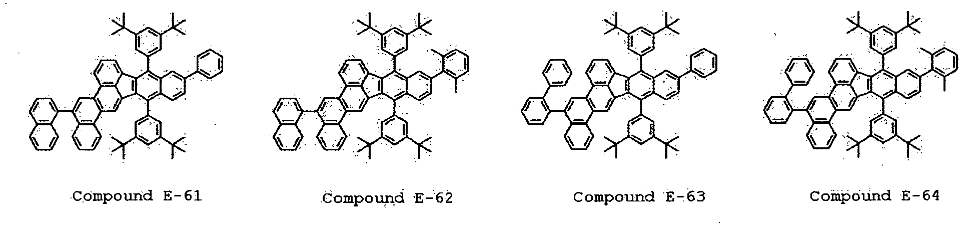

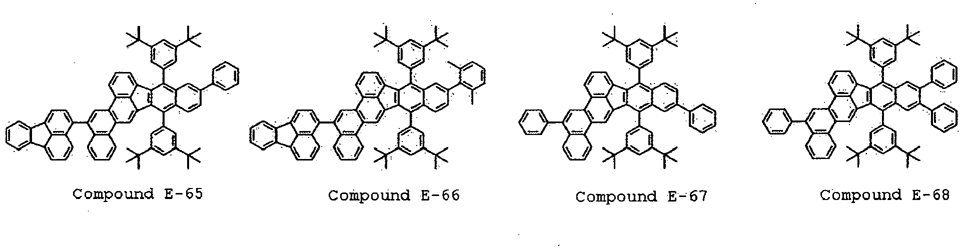

Next, the organic light-emitting device of the present invention will be described in detail.

The organic light-emitting device of the present invention includes an anode and a cathode, and an organic compound layer that is disposed between the anode and the cathode.

The organic compound layer that is provided between the anode and the cathode of the organic light-emitting device may be either a single layer or a plurality of layers.

As a specific example of layer constitution, a first layer constitution is a constitution in which a substrate, an anode, a light-emitting layer and a cathode are stacked in the mentioned order.

A second layer constitution is a constitution in which a substrate, an anode, a hole transport layer, an electron transport layer, and a cathode are stacked in the mentioned order. A case in which a light-emitting layer is a hole transport layer and an electron transport layer is also included. In this case, light emission occurs at an interface of the two layers.

A third layer constitution is a constitution in which a substrate, an anode, a hole transport layer, a light-emitting layer, an electron transport layer and a cathode are stacked in the mentioned order.

As an example of the organic light-emitting device, the third layer constitution is shown in FIG. 1. FIG. 1 illustrates an organic light-emitting device 30. In the figure, reference numeral 1 denotes a substrate such as a glass plate, 2 denotes an anode, 5 denotes a hole transport layer, 3 denotes a light-emitting layer, 6 denotes an electron transport layer, and 4 denotes a cathode. The anode 2 is, for example, a reflection side electrode and is formed of a reflective member or a transparent electrode having a reflective member. In the case where the anode 2 is a reflection side electrode, the cathode 4 is, for example, a light extracting electrode. In this case, the cathode is a light-transmissive electrode such as ITO.

The benzoindenochrysene compound of the present invention can be contained in any one of the layers that are provided in the layer constitutions described above.

The constitution of an organic light-emitting device which uses the benzoindenochrysene compound of the present invention is not limited to those described above. For example, other various layer constitutions are available and a constitution in which an insulating layer, an adhesive layer or an interfering layer is provided at an interface between an electrode and an organic compound layer, a constitution in which a hole transport layer consists of two layers having different ionization potentials, a constitution in which an light-emitting layer has a stacked structure with at least two layers, etc. can be mentioned.

The benzoindenochrysene compound of the present invention which is contained in one layer may be a single species or a plurality of species.

Among the layers that are included in the specific layer constitutions described above, the benzoindenochrysene compound of the present invention is preferably contained in any one layer of a light-emitting layer, a hole transport layer and an electron transport layer. More preferably, it is contained in a light-emitting layer.

When the benzoindenochrysene compound of the present invention is contained in a light-emitting layer, it is possible that the light-emitting layer consists of only the benzoindenochrysene compound of the present invention. However, preferably, it consists of a host and a guest. Herein, the guest is a compound which is responsible for major light emission in a light-emitting layer. The host is a compound which is present as a matrix around a guest in a light-emitting layer, and it is mainly responsible for carrier transport and supply of excitation energy to a guest. When a light-emitting layer consists of a host and a guest, the benzoindenochrysene compound of the present invention is preferably used as a guest. As a result, the emission efficiency of the organic light-emitting device is improved and light emission with high luminance can be maintained for a long period of time.

Incidentally, use of the benzoindenochrysene compound of the present invention is not limited to a material for constituting a light-emitting layer. For example, it can be used as a constitutional material for a hole transport layer, an electron transport layer, a hole blocking layer, an electron blocking layer, a hole injection layer, an electron injection layer and the like.

The organic light-emitting device of the present invention uses the benzoindenochrysene compound of the present invention as a constitution material of a light-emitting layer, in particular. However, if necessary, a low molecular weight type and a polymer type hole transport compound, light-emitting compound, electron transport compound and the like that are hitherto known in the field can be also used together.

Herein below, examples of such compounds are given.

As for a hole injection transport material, a material which is suitable for easy hole injection from an anode and has a high hole transporting property to transport the injected hole to a light-emitting layer is preferable. Examples of a low molecular weight type and a polymer type material having a hole injection transporting property include, but not limited to, a triarylamine derivative, a phenylene diamine derivative, a stilbene derivative, a phthalocyanin derivative, a porphyrin derivative, poly(vinylcarbazole), poly(thiophene) and other conductive polymers.

As for a light-emitting material mainly associated with light-emitting function, in addition to the benzoindenochrysene compound of the present invention, a fused ring compound (for example, a fluorene derivative, a pyrene derivative, a tetracene derivative, 9,10-diphenylanthracene derivative, lubrene and the like), a quinacridone derivative, a coumarine derivative, a stilbene derivative, an organoaluminum complex such as tris(8-quinolinolate)aluminum, an organoberyllium complex, and a polymer derivative such as a poly(phenylenevinylene) derivative, a poly(fluorene) derivative, and a poly(phenylene) derivative can be mentioned, but not limited thereto.

As for an electron injection transport material, any one that is suitable for easy electron injection from a cathode and can transport the injected electron to a light-emitting layer can be selected, and it is selected in consideration of balance with carrier transporting property of a hole transport material, etc. Examples of a material having an electron injection transporting property include, but not limited to, an oxadiazole derivative, an oxazole derivative, a pyrazine derivative, a triazole derivative, a triazine derivative, a quinoline derivative, a quinoxaline derivative, a phenanthroline derivative, an organoaluminum complex and the like.

As for a material constituting an anode, those having as large a work function as possible are preferred. Examples thereof include a metal element such as gold, platinum, silver, copper, nickel, palladium, cobalt, selenium, vanadium, and tungsten, or alloys of these elements, or a metal oxide such as tin oxide, zinc oxide, indium oxide, indium tin oxide (ITO), and zinc indium oxide. In addition, a conductive polymer such as polyaniline, polypyrrole, and polythiophene can be also used. These electrode materials can be used alone or in combination of two or more. In addition, the anode may consist of one layer or a plurality of layers.

As for a material constituting a cathode, those having as small a work function as possible are preferred. Examples thereof include an alkali metal such as lithium, an alkali earth metal such as calcium, and an elemental metal such as aluminum, titanium, manganese, silver, lead, and chromium. An alloy combining these metal elements can be also used. For example, magnesium-silver, aluminum-lithium, aluminum-magnesium and the like can be used. A metal oxide such as indium tin oxide (ITO) can be also used. These electrode materials can be used alone or in combination of two or more. In addition, the cathode may consist of one layer or a plurality of layers.

The substrate used in the organic light-emitting device of the present invention is not particularly limited, but an opaque substrate such as a metal substrate, and a ceramic substrate or a transparent substrate such as glass, quartz, and a plastic sheet can be used. In addition, a color filter film, a fluorescent color conversion filter film, a dielectric reflection film and the like can be used for the substrate to control the emitted light.

Incidentally, the produced device may be provided with a protective layer or an encapsulating layer for the purpose of preventing the device from contacting oxygen or moisture, and the like, for example. Examples of the protective layer include a diamond thin film, an inorganic material film made of, for example, a metal oxide or a metal nitride, a polymer film such as fluororesin, polyethylene, silicone resin, or polystyrene resin, and a photocurable resin and the like. In addition, the device may be covered with glass, a gas impermeable film, a metal, or the like, and the device itself can be packaged with an appropriate encapsulating resin.

It is also possible to make a thin film transistor (TFT) on a substrate and produce the organic light-emitting device of the present invention so as to be connected thereto.

In addition, with regard to the direction of extracting light from the device, both a bottom emission configuration (i.e., a configuration in which light is extracted from a substrate side) and a top emission configuration (i.e., a configuration in which light is extracted from the side opposite to the substrate side) are available.

With respect to the organic light-emitting device of the present invention, an organic compound layer including a layer which contains the benzoindenochrysene compound of the present invention is formed by the processes described below. In general, a thin film is formed by a vacuum evaporation process, an ionized evaporation process, a sputtering process, or a plasma process, or by dissolving a film material in an appropriate solvent and subjecting the solution to a known application method (such as a spin coating process, a dipping process, a casting process, an LB process, or an ink jet process). In this case, when a layer is formed by a vacuum evaporation process or by a solution coating process, crystallization and the like hardly occurs and the stability over time is excellent. Furthermore, when a film is formed by the coating method, a film may be formed by additionally using an appropriate binder resin.

Examples of the binder resin include, but are not limited to, a polyvinyl carbazole resin, a polycarbonate resin, a polyester resin, an ABS resin, an acrylic resin, a polyimide resin, a phenol resin, an epoxy resin, a silicone resin, a urea resin and the like. Furthermore, these binder resins are used in the form of a homopolymer or a copolymer. Moreover, they can be used alone or in combination of two or more. Still furthermore, if required, a known additive such as a plasticizer, an antioxidant, and a UV absorber may be used in combination with the binder resin.

### (Examples)

Herein below, the present invention will be described more specifically by way of Examples. However, the present invention is not limited to these Examples.

### (Example 1)

### Synthesis of Exemplified Compound B-1

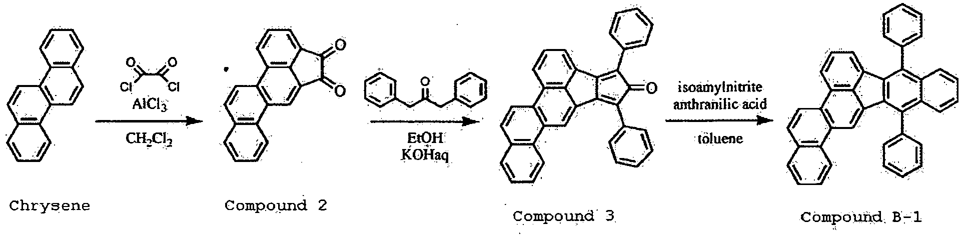

### (1) Synthesis of Compound 2

The following reagents and solvent were placed in a reaction vessel.
Chrysene: 20.0 g (87.6 mmol)
Aluminum chloride: 46.7 g (350 mmol)
Dichloromethane: 400 mL

Next, after establishing a nitrogen atmosphere in the reaction vessel, the reaction solution was cooled to -78°C and 55.6 g (438 mmol) of oxalyl chloride was added dropwise thereto with stirring at that temperature. Then, while maintaining the liquid temperature at -78°C, the reaction solution was stirred for 30 minutes and warmed to room temperature over 2 hours. Next, the reaction solution was poured into 4L of ice water with stirring and a resulting solid was separated by filtration. Then, the solid was dispersed and washed with 100 mL of methanol. After washing, the solid was vacuum dried with heating to give 21.5 g (yield: 87%) of Compound 2.

### (2) Synthesis of Compound 3

The following reagents and solvent were placed in a reaction vessel.
Compound 2: 2 g (7.1 mmol)
1,3-Diphenylpropan-2-one: 1.5 g (7.1 mmol)
Ethanol: 100 mL

Next, 25 mL of 2M aqueous potassium hydroxide solution was added dropwise to the reaction solution. Then, after heating the reaction solution to 75°C, the reaction solution was stirred for 2 hours at that temperature. Subsequently, the precipitate formed when cooling the reaction solution was filtered. The precipitate was washed with ethanol and water sequentially, and dried to give 3.2 g (7.1 mmol) of Compound 3.

### (3) Synthesis of Exemplified Compound B-1

The following reagents and solvent were placed in a reaction vessel.
Compound 3: 3 g (6.6 mmol)
Toluene: 300 mL
Anthranillic acid: 950 mg (6.9 mmol)
Isoamyl nitrite: 850 µl (6.9 mmol)

Next, the reaction solution was heated to 85°C and stirred for 5 hours at that temperature. Then, after cooling the reaction solution, liquid-liquid separation was carried out with 1M aqueous hydrochloric acid solution, aqueous sodium hydrocarbonate solution, and aqueous sodium chloride solution sequentially and the organic layer was recovered. After drying the organic layer over sodium sulfate, the solvent was evaporated under reduced pressure to give a crude product. The crude product was purified with column chromatography (chromatography gel: BW300 (trade name; manufactured by FUJI SILYSIA CHEMICAL LTD.); developing solvent: toluene), and recrystallized from toluene to give 2.7 g (3 mmol) of Exemplified Compound B-1 (yield: 80.0%).

Twenty four protons were assigned according to ¹H-NMR. ¹H-NMR (CDCl₃) : δ (ppm) = 8.57 (d, 1H), 8.43 ppm (d, 1H), 8.03 ppm (d, 1H), 7.95 - 7.90 ppm (m, 2H), 7.87 - 7.80 ppm (m, 4H), 7.78 ppm (t, 1H), 7.72 - 7.54 ppm (m, 10H), 7.50 ppm (t, 1H), 7.47 - 7.40 ppm (m, 2H), 6.73 ppm (d, 1H)

From MALDI-TOF MS (Matrix Assisted Ionization - Time of Flight Mass Spectrometry), a peak of m/z = 504 was obtained, and Exemplified Compound B-1 was identified.

### (Example 2)

### Synthesis of Exemplified Compound E-32

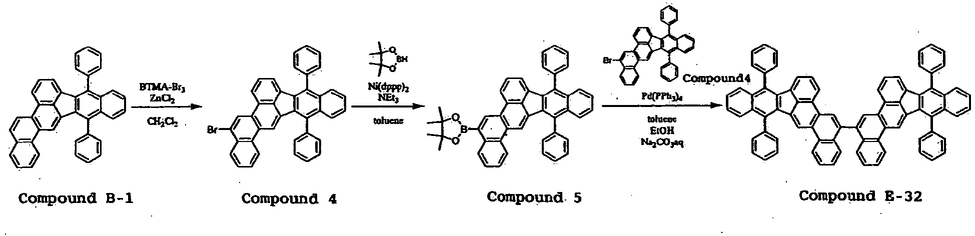

### (1) Synthesis of Compound 4

The following reagents and solvent were placed in a reaction vessel.
Compound B-1 : 1 g (2.0 mmol)
BTMA-Br₃ : 0.73 g (2.0 mmol)
Zinc chloride: 0.3 g (2.2 mmol)
Methylene chloride: 80 mL

Next, the reaction solution was stirred at room temperature for 10 hours. Next, after adding aqueous sodium sulfite solution, liquid-liquid separation with water was carried out and the organic layer was recovered. After drying the organic layer over sodium sulfate, the solvent was evaporated under reduced pressure to give a crude product. The crude product was recrystallized from toluene to give 1.1 g of Compound 4 (yield: 95.0%).

### (2) Synthesis of Compound 5

The following reagent and solvent were placed in a reaction vessel.
Compound 4: 0.5 g (0.86 mmol)
Toluene: 25 mL

Next, after introducing nitrogen gas into the reaction vessel and replacing the inside atmosphere of the reaction system with nitrogen atmosphere, the following reagents were further added.
4,4,5,5-Tetramethyl-[1,3,2] dioxaborolane: 350 µl (2.6 mmol)
Ni(dppp) Cl₂ : 70 mg (0.13 mmol)
Triethylamine : 10 mL (4.0 mmol)

Next, the reaction solution was heated to 105°C and stirred for 8 hours at that temperature. Then, after cooling the reaction solution, liquid-liquid separation with water was carried out and the organic layer was recovered. After drying the organic layer over sodium sulfate, the solvent was evaporated under reduced pressure to give a crude product. The crude product was purified with column chromatography (chromatography gel: BW300 (trade name; manufactured by FUJI SILYSIA CHEMICAL LTD.); developing solvent: heptane/toluene = 1/4), and recrystallized from toluene to give 0.23 g of Compound 5 (yield: 41.3%).

### (3) Synthesis of Exemplified Compound E-32

The following reagents and solvents were placed in a reaction vessel.
Compound 4: 102 mg (0.17 mmol)
Compound 5: 100 mg (0.16 mmol)
Toluene: 10 mL
Ethanol: 5 mL
2M Aqueous sodium carbonate solution: 10 mL

Next, after introducing nitrogen gas into the reaction vessel and replacing the inside atmosphere of the reaction system with nitrogen atmosphere, 8 mg of Pd(PPh₃)₄ was added. Next, the reaction solution was heated to 75°C and stirred for 6 hours at that temperature. After cooling the reaction solution, liquid-liquid separation with water was carried out and the organic layer was recovered. After drying the organic layer over sodium sulfate, the solvent was evaporated under reduced pressure to give a crude product. The crude product was purified with column chromatography (chromatography gel: BW300 (trade name; manufactured by FUJI SILYSIA CHEMICAL LTD.); developing solvent: heptane/toluene = 1/4), and recrystallized from toluene to give 90 g (0.14 mmol) of Exemplified Compound E-32 (yield: 69.4%).

Twenty four protons were assigned according to ¹H-NMR. ¹H-NMR (CDCl₃) : δ (ppm) = 8.77 (s, 2H), 8.42 ppm (d, 2H), 8.18 ppm (d, 2H), 8.01 ppm (s, 2H), 7.95 - 7.87 ppm (m, 6H), 7.87 - 7.76 ppm (m, 6H), 7.76 - 7.64 ppm (m, 12H), 7.61 ppm (t, 2H), 7.53 - 7.45 ppm (m, 8H), 7.35 ppm (t, 2H), 6.78 ppm (d, 2H))

From MALDI-TOF MS (Matrix Assisted Ionization - Time of Flight Mass Spectrometry), a peak of m/z = 1007 was obtained, and Exemplified Compound E-32 was identified.

### (Example 3)

### Synthesis of Exemplified Compound E-28

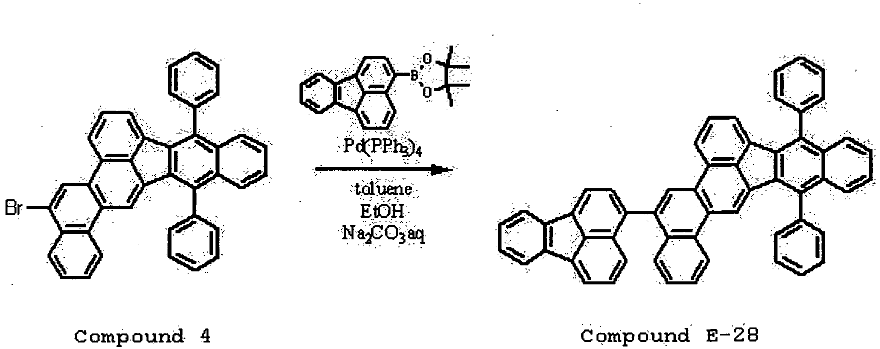

The following reagents and solvents were placed in a reaction vessel.
Compound 4: 120 mg (0.21 mmol)
3-Fluoranthenyl boronic acid: 36 mg
Toluene : 10 mL
Ethanol : 5 mL
2M Aqueous sodium carbonate solution: 10 mL

Next, after introducing nitrogen gas into the reaction vessel and replacing the inside atmosphere of the reaction system with nitrogen atmosphere, 8 mg of Pd(PPh₃)₄ was further added.

Next, the reaction solution was heated to 75°C and stirred for 6 hours at that temperature. After cooling the reaction solution, liquid-liquid separation with water was carried out and the organic layer was recovered. After drying the organic layer over sodium sulfate, the solvent was evaporated under reduced pressure to give a crude product. The crude product was purified with column chromatography (chromatography gel: BW300 (trade name; manufactured by FUJI SILYSIA CHEMICAL LTD.), developing solvent: heptane/toluene = 1/4), and recrystallized from toluene to give 90 g (0.14 mmol) of Exemplified Compound E-28 (yield: 69.4%).

Twenty four protons were assigned according to ¹H-NMR. ¹H-NMR (CDCl₃) : δ (ppm) = 8.70 (s, 1H), 8.42 ppm (d, 1H), 8.18 ppm (d, 1H), 8.12 ppm (s, 1H), 8.05 - 7.93 ppm (m, 4H), 7.93 - 7.86 ppm (m, 3H), 7.86 - 7.61 ppm (m, 12H), 7.53 - 7.40 ppm (m, 8H), 6.78 ppm (d, 1H)

From MALDI-TOF MS (Matrix Assisted Ionization - Time of Flight Mass Spectrometry), a peak of m/z = 630 was obtained, and Exemplified compound E-28 was identified.

### (Example 4)

### Synthesis of Exemplified Compound E-13

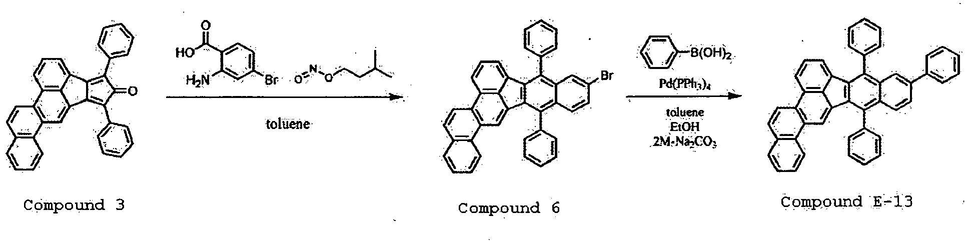

### (1) Synthesis of Compound 6

The following reagents and solvent were placed in a reaction vessel.
Compound 3: 4 g (8.8 mmol)
Toluene: 400 mL
4-Bromoanthranillic acid: 1890 mg (8.8 mmol)
Isoamyl nitrite: 1030 mg (8.8 mmol)

Next, the reaction solution was heated to 85°C and stirred for 4 hours at that temperature. After cooling the reaction solution, liquid-liquid separation was carried out with 1M aqueous hydrochloric acid solution, aqueous sodium hydrogen carbonate solution, and aqueous sodium chloride solution sequentially and the organic layer was recovered. After drying the organic layer over sodium sulfate, the solvent was evaporated under reduced pressure to give a crude product. The crude product was purified with column chromatography (chromatography gel: BW300 (trade name; manufactured by FUJI SILYSIA CHEMICAL LTD.); developing solvent: toluene), and recrystallized from toluene to give 3.1 g (5.3 mmol) of Compound 6 (yield: 60.2%).

From MALDI-TOF MS (Matrix Assisted Ionization - Time of Flight Mass Spectrometry), a peak of m/z = 584 was obtained, and Compound 6 was identified.

### (2) Synthesis of Exemplified Compound E-13

The following reagents and solvents were placed in a reaction vessel.
Compound 6: 500 mg (0.86 mmol)
Phenyl boronic acid: 125 mg (1.03 mmol)
Toluene : 30 mL
Ethanol : 15 mL
2M Aqueous sodium carbonate solution: 30 mL

Next, after replacing the inside atmosphere of the reaction system with nitrogen atmosphere, 50 mg of Pd(PPh₃)₄ was added. Then, the reaction solution was heated to 85°C and stirred for 6 hours at that temperature. After cooling the reaction solution, liquid-liquid separation with water was carried out and the organic layer was recovered. After drying the organic layer over sodium sulfate, the solvent was evaporated under reduced pressure to give a crude product. The crude product was purified with column chromatography (chromatography gel: BW300 (trade name; manufactured by FUJI SILYSIA CHEMICAL LTD.); developing solvent: heptane/toluene =1/4), and recrystallized from toluene to give 441 g (0.76 mmol) of Compound E-13 (yield: 88.3%).

Twenty four protons were assigned according to ¹H-NMR. ¹H-NMR (CDCl₃) : δ (ppm) = 8.59 (d, 1H), 8.45 ppm (d, 1H), 8.03 ppm (d, 1H), 7.97 - 7.91 ppm (m, 2H), 7.90 - 7.82 ppm (m, 5H), 7.76 - 7.54 ppm (m, 13H), 7.51 ppm (t, 1H), 7.45 - 7.37 ppm (m, 2H), 7.37 - 7.31 (m, 1H), 6.75 ppm (d, 1H)

From MALDI-TOF MS (Matrix Assisted Ionization - Time of Flight Mass Spectrometry), a peak of m/z = 581 was obtained, and Exemplified Compound E-13 was identified.

### (Example 5)

An organic light-emitting device having a layer constitution as described in the third layer constitution above and shown in FIG. 1 was produced according to the following method.

By forming an indium tin oxide (ITO) film on a glass substrate using a sputtering method, an anode was formed. The film thickness of the anode 2 was 120 nm. Next, the substrate on which the ITO electrode has been formed was ultrasonically cleaned sequentially with acetone and isopropyl alcohol (IPA), and then washed with boiled IPA. After drying, the substrate was cleaned with UV/ozone. The substrate treated according to the method described above was used as a transparent conductive support substrate.

Next, Compound 7 shown below and chloroform were mixed with each other to prepare a chloroform solution having 0.1 weight% concentration. Then, to the anode, the chloroform solution obtained above was dropped and coated, and a film was formed by a spin coating method to give a hole transport layer. At this time, the thickness of the hole transport layer was 20 nm.

Next, the substrate having the layers up to and including the hole transport layer formed thereon was transferred to a vacuum chamber. After adjusting the inside pressure of the vacuum chamber to 10⁻⁵Pa, with vacuum evaporation based on resistive heating, continuous film formation of an organic compound layer and an electrode layer on the hole transport layer was carried out. Specifically, Compound 8 as a host shown below and Exemplified Compound E-28 as a guest were co-evaporated in a weight concentration ratio of 95 to 5 to form a light-emitting layer. At this time, the film thickness of the light-emitting layer was 20 nm. Thereafter, film formation of Compound 8 shown below was carried out to form an electron transport layer. At this time, the film thickness of the electron transport layer was 40 nm. Then, LiF film formation was carried out to form a first metal electrode layer. At this time, the film thickness of the first metal electrode layer was 0.5 nm. Next, Al film formation was carried out to form a second metal electrode layer. At this time, the film thickness of the second metal electrode layer was 150 nm. As a result, an organic light-emitting device was obtained.

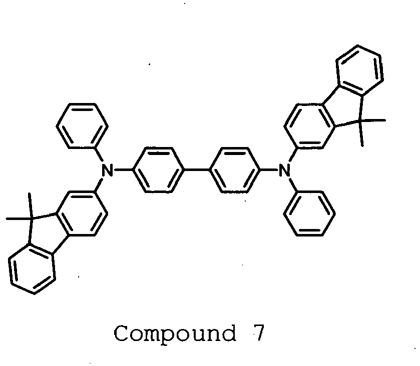

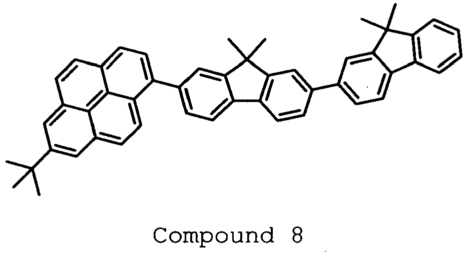

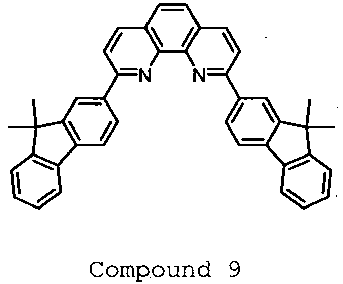

The characteristics of the thus obtained organic light-emitting device were then measured and evaluated. Specifically, the current/voltage characteristics of the device were measured by using Pico-Amp Meter 4140B (manufactured by Hewlett-Packard Development Company L.P.) and the emission luminance of the device was measured by BM7 (manufactured by TOPCON CORPORATION). As a result of the measurement, it was found that the organic light-emitting device of the present example showed blue light emission having CIE standard colorimetric system (chromaticity coordinates) of (x, y) = (0.140, 0.189) when the emission luminance was 2000 cd/m². Furthermore, the emission efficiency was 6.9 cd/A and the external quantum yield was 4.8%. Moreover, when a voltage was continuously applied for 100 hours to the device in a nitrogen atmosphere, favorable light emission was observed continuously.

### (Examples 6 to 14)

Organic light-emitting devices were prepared by following the same procedure as in Example 5 with the exception that the host and the guest and their mixing ratio by weight in the light-emitting layer were changed to those shown in Table 4 below. With the resulting devices, the evaluation was carried out in the same manner as in Example 5. The results are shown in Table 4.

**(Table 4)**

| | Light-emitting layer | | CIE Standard colorimetric system | | Emission efficiency | External quantum yield |
|---|---|---|---|---|---|---|
| | Host | Guest | x | y | [cd/A] | [%] |
| Example 6 | Compound 8 (95 weight %) | E-30 (5 weight %) | 0.140 | 0.172 | 7.3 | 5.5 |
| Example 7 | Compound 8 (90 weight %) | E-30 (10 weight %) | 0.141 | 0.190 | 7.6 | 5.5 |
| Example 8 | Compound 8 (95 weight %) | E-28 (5 weight %) | 0.140 | 0.189 | 6.9 | 4.8 |
| Example 9 | Compound 8 (95 weight %) | E-36 (5 weight %) | 0.140 | 0.189 | 6.5 | 4.6 |
| Example 10 | Compound 8 (90 weight %) | E-36 (10 weight %) | 0.141 | 0.215 | 8.1 | 5.3 |
| Example 11 | Compound 8 (95 weight %) | B-7 (5 weight %) | 0.144 | 0.126 | 5.1 | 4.7 |
| Example 12 | Compound 8 (90 weight %) | B-7 (10 weight %) | 0.145 | 0.138 | 4.3 | 3.7 |
| Example 13 | Compound 8 (95 weight %) | E-25 (5 weight %) | 0.143 | 0.121 | 5.2 | 5.0 |
| Example 14 | Compound 8 (90 weight %) | E-25 (10 weight %) | 0.142 | 0.124 | 5.2 | 4.9 |

As shown in Table 4 above, the organic light-emitting devices that were prepared according to Examples 6 to 14 all exhibited blue light emission. Furthermore, when a voltage was continuously applied for 100 hours to each of the devices in a nitrogen atmosphere, favorable light emission was observed continuously.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

This application claims the benefit of Japanese Patent Application No. 2008-314391, filed December 10, 2008.

## Claims

1. A benzoindenochrysene compound represented by the general formula (1): 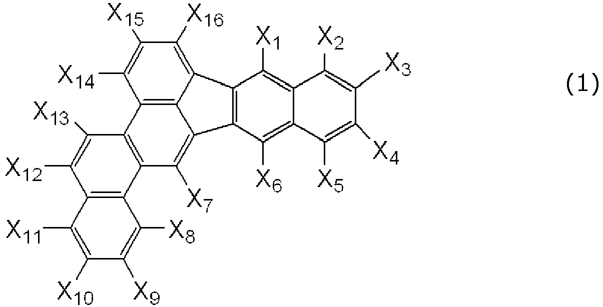 wherein X₁, X₂, X₃, X₄, X₅, X₆, X₇, X₈, X₉, X₁₀, X₁₁, X₁₂, X₁₃, X₁₄, X₁₅, and X₁₆ each represent, independently of one another, a hydrogen atom, a halogen atom, a cyano group, a nitro group, a substituted or unsubstituted alkyl group, a substituted or unsubstituted alkoxy group, a substituted or unsubstituted alkenyl group, a substituted or unsubstituted alkynyl group, a substituted or unsubstituted aralkyl group, a substituted amino group, a substituted or unsubstituted aryl group, a substituted or unsubstituted heterocyclic group, or a substituted or unsubstituted aryloxy group.

2. The benzoindenochrysene compound according to claim 1, which is represented by the general formula (2): 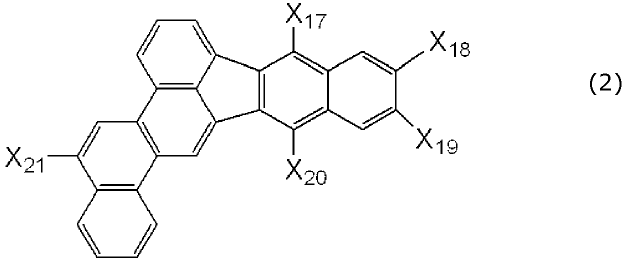 wherein X₁₇, X₁₈, X₁₉, X₂₀, and X₂₁ each represent, independently of one another, a hydrogen atom or a substituted or unsubstituted aryl group.

3. A benzoindenochrysene compound according to claim 1, which is represented by the general formula (2): 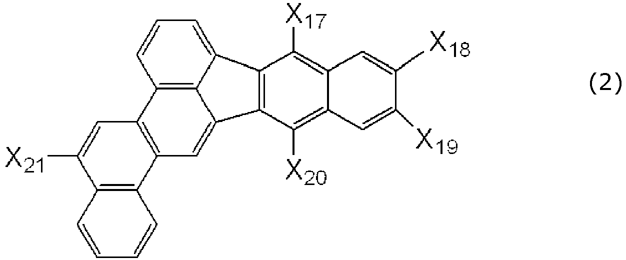
wherein X₁₇ and X₂₀ each represent a hydrogen atom or a substituted or unsubstituted phenyl group, and the substituted phenyl group may have any of the group consisting of a methyl group, an ethyl group, an n-propyl group, an iso-propyl group, and a tert-butyl group;
at least one of X₁₈ and X₁₉ is a phenyl group or a naphthyl group, and one of X₁₈ and X₁₉, when being neither a phenyl group nor a naphthyl group, is a hydrogen atom, and the phenyl group or the naphthyl group may have a substituent selected from the group consisting of a methyl group, an ethyl group, an n-propyl group, an iso-propyl group, and a tert-butyl group; and X₂₁ is a substituent selected from the group consisting of a naphthyl group, a phenanthryl group, a biphenyl group, a terphenyl group, a binaphthyl group, a fluoranthenyl group, a benzofluoranthenyl group, and an indeno chrysenyl group, and the substituent may have a methyl group or a phenyl group.

4. The benzoindenochrysene compound according to claim 3, wherein X₂₁ is a substituent selected from the group consisting of a fluoranthenyl group, a benzofluoranthenyl group, and an indeno chrysenyl group, and the substituent may have a methyl group or a phenyl group.

5. The benzoindenochrysene compound according to claim 4, wherein the benzoindenochrysene skeleton of the general formula (2) is bonded to position 3 or 8 of the fluoranthenyl group, and wherein the benzoindenochrysene skeleton of the general formula (2) is bonded to position 3 of the benzofluoranthenyl group.

6. An organic light-emitting device comprising;
an anode and a cathode and,
an organic compound layer disposed between the anode and the cathode,
wherein at least one layer of the organic compound layer comprises at least one kind of the benzoindenochrysene compound set forth in claims 1-5.

7. The organic light-emitting device according to claim 6, wherein the benzoindenochrysene compound is contained in a light-emitting layer.

8. The organic light-emitting device according to claim 7, wherein the light-emitting layer further comprises at least one of a pyrene derivative, an organoaluminum complex, and an organoberyllium complex.

9. The organic light-emitting device according to claim 6, wherein the organic compound layer includes a light-emitting layer, the light emitting layer comprises a host and a guest, and the benzoindenochrysene compound is the guest.

10. A full-color display comprising the organic light-emitting device set forth in any one of claims 6-9, wherein the benzoindenochrysene compound emits blue light.

## Patentansprüche

1. Benzoindenochrysenverbindung, die durch die allgemeine Formel (1) dargestellt ist: 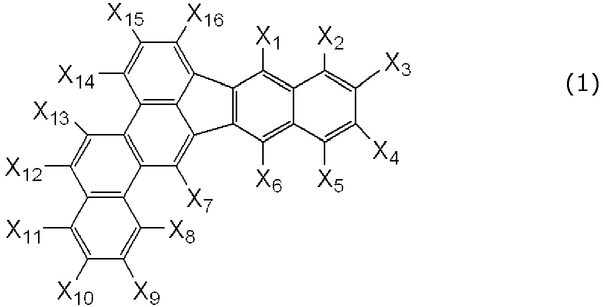 wobei X₁, X₂, X₃, X₄, X₅, X₆, X₇, X₈, X₉, X₁₀, X₁₁, X₁₂, X₁₃, X₁₄, X₁₅ und X₁₆ jeweils unabhängig voneinander ein Wasserstoffatom, ein Halogenatom, eine Cyanogruppe, eine Nitrogruppe, eine substituierte oder unsubstituierte Alkylgruppe, eine substituierte oder unsubstituierte Alkoxygruppe, eine substituierte oder unsubstituierte Alkenylgruppe, eine substituierte oder unsubstituierte Alkinylgruppe, eine substituierte oder unsubstituierte Aralkylgruppe, eine substituierte Aminogruppe, eine substituierte oder unsubstituierte Arylgruppe, eine substituierte oder unsubstituierte heterocyclische Gruppe oder eine substituierte oder unsubstituierte Aryloxygruppe darstellen.

2. Benzoindenochrysenverbindung nach Anspruch 1, welche durch die allgemeine Formel (2) dargestellt ist: 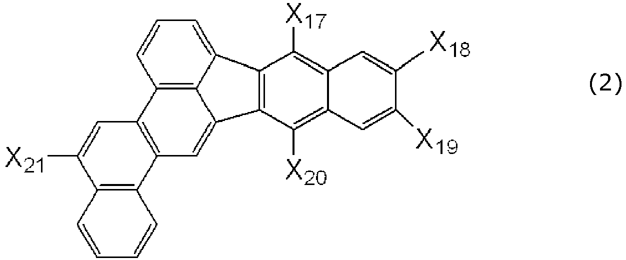 wobei X₁₇, X₁₈, X₁₉, X₂₀ und X₂₁ jeweils unabhängig voneinander ein Wasserstoffatom oder eine substituierte oder unsubstituierte Arylgruppe darstellen.

3. Benzoindenochrysenverbindung nach Anspruch 1, welche durch die allgemeine Formel (2) dargestellt ist: 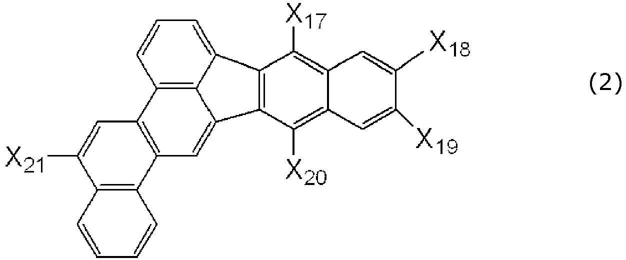
wobei X₁₇ und X₂₀ jeweils ein Wasserstoffatom oder eine substituierte oder unsubstituierte Phenylgruppe darstellen, und die substituierte Phenylgruppe eines aus der Gruppe, die aus einer Methylgruppe, einer Ethylgruppe, einer n-Propylgruppe, einer iso-Proplygruppe und einer tert-Butylgruppe besteht, aufweisen kann;
zumindest eines aus X₁₈ und X₁₉ eine Phenylgruppe oder eine Naphthylgruppe ist, und eines aus X₁₈ und X₁₉, wenn es weder eine Phenylgruppe noch eine Naphthylgruppe ist, ein Wasserstoffatom ist, und die Phenylgruppe oder die Naphthylgruppe einen Substituenten aufweisen kann, der aus der Gruppe bestehend aus einer Methylgruppe, einer Ethylgruppe, einer n-Propylgruppe, einer iso-Propylgruppe und einer tert-Butylgruppe ausgewählt ist; und
X₂₁ ein Substituent ist, der aus der Gruppe bestehend aus einer Naphthylgruppe, einer Phenanthrylgruppe, einer Biphenylgruppe, einer Terphenylgruppe, einer Binaphthylgruppe, einer Fluoranthenylgruppe, einer Benzofluoranthenylgruppe und einer Indenochrysenylgruppe ausgewählt ist, und der Substituent eine Methylgruppe oder eine Phenylgruppe aufweisen kann.

4. Benzoindenochrysenverbindung nach Anspruch 3, wobei X₂₁ ein Substituent ist, der aus der Gruppe bestehend aus einer Fluoranthenylgruppe, einer Benzofluoranthenylgruppe und einer Indenochrysenylgruppe ausgewählt ist, und der Substituent eine Methylgruppe oder Phenylgruppe aufweisen kann.

5. Benzoindenochrysenverbindung nach Anspruch 4, wobei das Benzoindochrysengerüst der allgemeinen Formel (2) an die Position 3 oder 8 der Fluoranthenylgruppe gebunden ist und wobei das Benzoindenochrysengerüst der allgemeinen Formel (2) an die Position 3 der Benzofluoranthenylgruppe gebunden ist.

6. Organische Licht-emittierende Vorrichtung, die umfasst;
eine Anode und eine Kathode und
eine organische-Verbindung-Schicht, die zwischen der Anode und der Kathode angeordnet ist,
wobei zumindest eine Schicht der organische-Verbindung-Schicht zumindest eine Art der Benzoindenochrysenverbindung nach Ansprüchen 1-5 umfasst.

7. Organische Licht-emittierende Vorrichtung nach Anspruch 6, wobei die Benzoindenochrysenverbindung in einer Licht-emittierenden Schicht enthalten ist.

8. Organische Licht-emittierende Vorrichtung nach Anspruch 7, wobei die Licht-emittierende Schicht ferner zumindest eines aus einem Pyrenderivat, einem Organoaluminiumkomplex und einem Organoberylliumkomplex umfasst.

9. Organische Licht-emittierende Vorrichtung nach Anspruch 6, wobei die organische-Verbindung-Schicht eine Licht-emittierende Schicht beinhaltet, wobei die Licht-emittierende Schicht einen Wirt und einen Gast umfasst und die Benzoindenochrysenverbindung der Gast ist.

10. Vollfarben-Anzeige, die die organische Licht-emittierende Vorrichtung nach einem der Ansprüche 6-9 umfasst, wobei die Benzoindenochrysenverbindung blaues Licht emittiert.

## Revendications

1. Dérivé de benzo-indénochrysène représenté par la formule générale (1) : 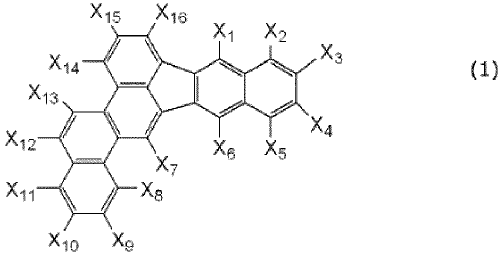 dans laquelle X₁, X₂, X₃, X₄, X₅, X₆, X₇, X₈, X₉, X₁₀, X₁₁, X₁₂, X₁₃, X₁₄, X₁₅ et X₁₆ représentent chacun, indépendamment les uns des autres, un atome d'hydrogène, un atome d'halogène, un groupe cyano, un groupe nitro, un groupe alkyle substitué ou non substitué, un groupe alkoxy substitué ou non substitué, un groupe alcényle substitué ou non substitué, un groupe alcynyle substitué ou non substitué, un groupe aralkyle substitué ou non substitué, un groupe amino substitué, un groupe aryle substitué ou non substitué, un groupe hétérocyclique substitué ou non substitué ou un groupe aryloxy substitué ou non substitué.

2. Dérivé de benzo-indénochrysène suivant la revendication 1, qui est représenté par la formule générale (2) : 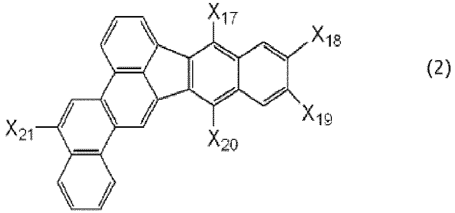 dans laquelle X₁₇, X₁₈, X₁₉, X₂₀ et X₂₁ représentent chacun, indépendamment les uns des autres, un atome d'hydrogène ou un groupe aryle substitué ou non substitué.

3. Dérivé de benzo-indénochrysène suivant la revendication 1, qui est représenté par la formule générale (2) : 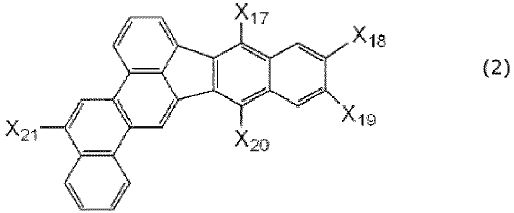
dans laquelle X₁₇ et X₂₀ représentent chacun un atome d'hydrogène ou un groupe phényle substitué ou non substitué, et le groupe phényle substitué peut avoir l'un quelconque du groupe consistant en un groupe méthyle, un groupe éthyle, un groupe n-propyle, un groupe isopropyle, et un groupe tertiobutyle ;
au moins l'un des X₁₈ et X₁₉ est un groupe phényle ou un groupe naphtyle, et l'un entre X₁₈ et X₁₉, en n'étant ni un groupe phényle ni un groupe naphtyle, est un atome d'hydrogène, et le groupe phényle ou le groupe naphtyle peut avoir un substituant choisi dans le groupe consistant en un groupe méthyle, un groupe éthyle, un groupe n-propyle, un groupe isopropyle et un groupe tertiobutyle ; et X₂₁ est un substituant choisi dans le groupe consistant en un groupe naphtyle, un groupe phénanthryle, un groupe biphényle, un groupe terphényle, un groupe binaphtyle, un groupe fluoranthényle, un groupe benzo-fluoranthényle et un groupe indéno-chrysényle, et le substituant peut avoir un groupe méthyle ou un groupe phényle.

4. Dérivé de benzo-indénochrysène suivant la revendication 3, dans lequel X₂₁ est un substituant choisi dans le groupe consistant en un groupe fluoranthényle, un groupe benzo-fluoranthényle et un groupe indéno-chrysényle, et le substituant peut avoir un groupe méthyle ou un groupe phényle.

5. Dérivé de benzo-indénochrysène suivant la revendication 4, dans lequel le squelette de benzo-indénochrysène de la formule générale (2) est lié à la position 3 ou 8 du groupe fluoranthényle et où le squelette de benzo-indénochrysène de la formule générale (2) est lié à la position 3 du groupe benzo-fluoranthényle.

6. Dispositif électroluminescent organique comprenant :
une anode et une cathode, et
une couche d'un composé organique disposé entre l'anode et la cathode,
dans lequel au moins une couche de la couche de composé organique comprend au moins un type du dérivé de benzo-indénochrysène indiqué dans les revendications 1 à 5.

7. Dispositif électroluminescent organique suivant la revendication 6, dans lequel le dérivé de benzo-indénochrysène est présent dans une couche électroluminescente.

8. Dispositif électroluminescent organique suivant la revendication 7, dans lequel la couche électroluminescente comprend en outre au moins l'un d'un dérivé de pyrène, d'un complexe d'organo-aluminium et d'un complexe d'organo-beryllium.

9. Dispositif électroluminescent organique suivant la revendication 6, dans lequel la couche de composé organique comporte une couche électroluminescente, la couche électroluminescente comprend un composé incluant et un composé inclus et le dérivé de benzo-indénochrysène est le composé inclus.

10. Dispositif d'affichage polychrome comprenant le dispositif électroluminescent organique indiqué dans l'une quelconque des revendications 6 à 9, dans lequel le dérivé de benzo-indénochrysène émet une lumière bleue.
